# EUROPEAN PATENT SPECIFICATION

(11) **EP 2 298 172 B1**
(45) Date of publication and mention of the grant of the patent: **20.06.2012**
(21) Application number: 10155544.9
(22) Date of filing: 04.03.2010
(51) Int. Cl.: A61B 5/151

(54) **Blood lancing device**
Blutlanzettenvorrichtung
Autopiqueur de sang

(30) Priority: 18.09.2009 TW 098131592; 06.11.2009 TW 098137767
(43) Date of publication of application: 23.03.2011
(73) Proprietor: EPS Bio Technology Corp., Hsinchu Science Park 300 (TW)
(72) Inventor: Lee, Chi-Nan, Hsin-chu City (TW)
(74) Representative: Lang, Christian

(56) References cited:
- EP-A1- 1 992 283
- EP-A2- 1 779 780
- DE-A1- 19 723 730
- US-A1- 2006 241 669
- US-A1- 2008 167 578

## Description

### Background of the invention (a) Field of the Invention

The present invention relates to a blood lancing device, and more particularly, to a blood lancing device whose lancet holder is not easy to be vibrated.

### (b) Description of the Related Art

People nowadays are more concerned about their health. As medical devices developed, blood lancing devices and blood glucose meters, which people may use to test their blood at home by themselves, are sold at the market. In testing operation, a blood lancing device is used to have a skin of a fingertip bleed, and then a blood glucose meter is used to test the blood.

According to conventional design, a conventional blood lancing device mainly comprises a lancet holder and a housing. A lancet is disposed in the lancet holder. The inner walls of the housing define a containing space in which the lancet holder is disposed. The lancet holder may be moved in the containing space and be optionally extended out of the housing to puncture a skin of a fingertip for collecting blood. According to another conventional design described in the document US2006/0241669, a lancing device may comprise a lancet carrier, at least one drive spring, a charging mechanism, and a release mechanism to provide a lancing device with a compact size and narrow profile.

### BRIEF SUMMARY OF THE INVENTION

One objective of an embodiment of the invention is to provide a blood lancing device in which vibration of the lancet holder is not easy to occur. Another objective of an embodiment of the invention is to provide a blood lancing device in which the friction force during move of the lancet holder may be reduced.
These objects are achieved by a blood lancing device according to claim 1. Preferred embodiments are subject matter of the dependent claims.

According to an embodiment of the invention, the blood lancing device comprises a housing, a lancet holder and at least a slide rail. The lancet holder is disposed in the housing and defines at least a through hole. The slide rail is fixed in the housing and is inserted into the through hole such that the lancet holder is movable along the slide rail. Since the through hole and the slide rail can limit the lateral movement of the lancet holder, compared with the conventional device, the blood lancing device according to an embodiment has the advantage that vibration of the lancet holder is not easy to occur.

According to an embodiment of the invention, the lancet holder comprises a body portion and at least a wing portion. The body portion is for containing a lancet, and the wing portion extends from the body portion and defines the through hole. It is preferred that the height of the wing portion is smaller than that of the body portion.

According to an embodiment of the invention, the blood lancing device further comprises a push structure. The push structure comprises a push-button and an extending portion. The push-button is exposed on the outside of the housing and the extending portion extends from the push-button toward the inside of the housing and defines a hole. The slide rail is inserted into the hole so that the extending portion is in contact with the wing portion. In one embodiment, the blood lancing device further comprises a resilient element. The slide rail is inserted into the resilient element, and the resilient element and the extending portion are disposed at the two opposite sides of the wing portion.

According to an embodiment of the invention, the blood lancing device further comprises a resilient component. The other slide rail is inserted into the resilient component and the resilient component is located between the housing and the other wing portion.

Other purposes and benefits of the invention can be further understood by the technical features disclosed in the above-mentioned embodiments. In order to provide better and more obvious understanding of the purposes, features, and benefits of the invention, the detailed descriptions of the embodiments according to the invention will be given in the following together with figures.

### Brief description of the drawings

FIG. 1 shows an exploded view of a blood lancing device according to an embodiment of the present invention;

FIG. 2A shows a schematic diagram for illustrating a lancet holder being disposed at a bottom cover of a blood lancing device according to an embodiment of the present invention;

FIG. 2B shows a side view of a blood lancing device according to an embodiment of the present invention;

FIG. 3 shows a grossly enlarged fragmentary view of the blood lancing device shown in FIG. 2A;

FIG. 4 shows a three dimensional view of a lancet holder according to an embodiment of the present invention.

### DETAILED DESCRIPTION OF THE INVENTION

The above mentioned and other technical contents, uniqueness, and effects of the invention can be illustrated more clearly by the following detailed descriptions together with the corresponding figures. The wording describing directions used in the following descriptions, such as: up, down, left, right, front, back or the like, indicates the directions with respect to the figure only. Therefore, the wording used to describe directions is for illustration but not to limit the scope of the invention.

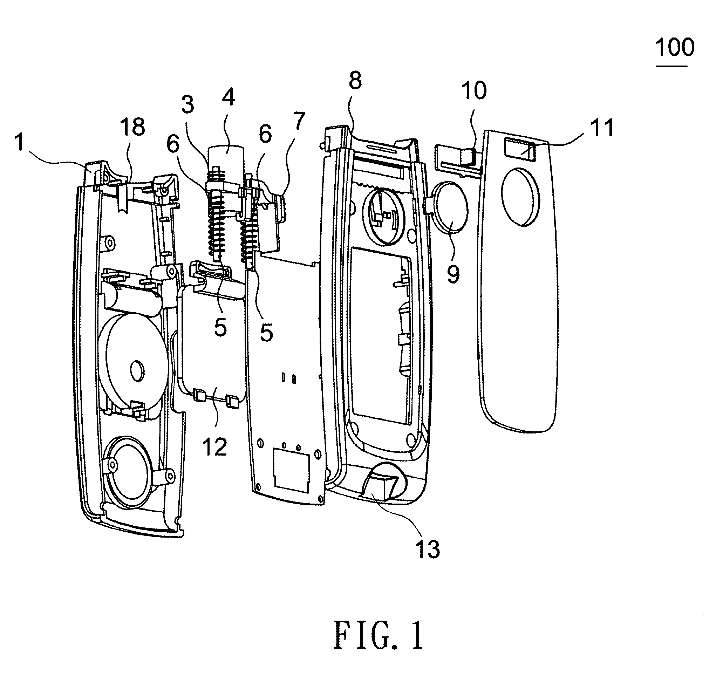

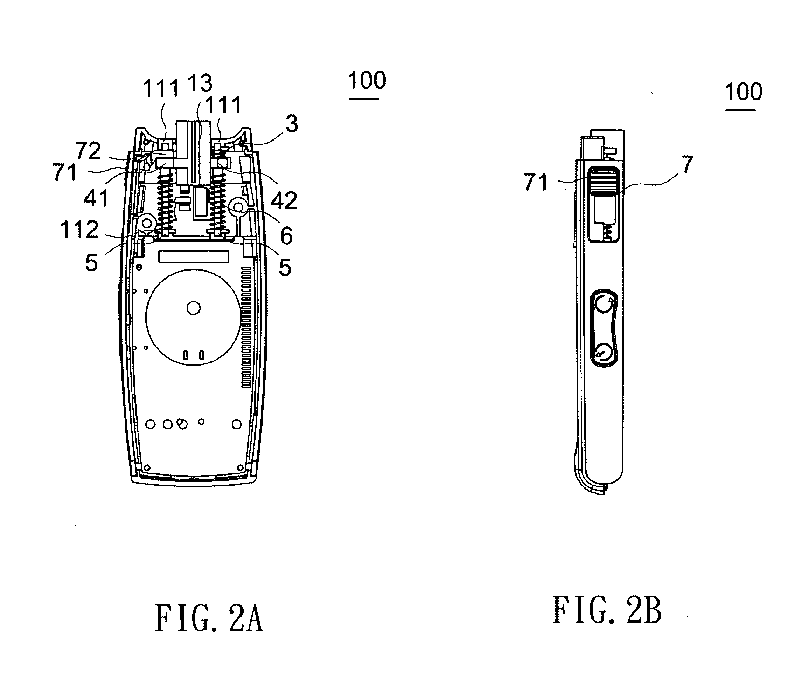

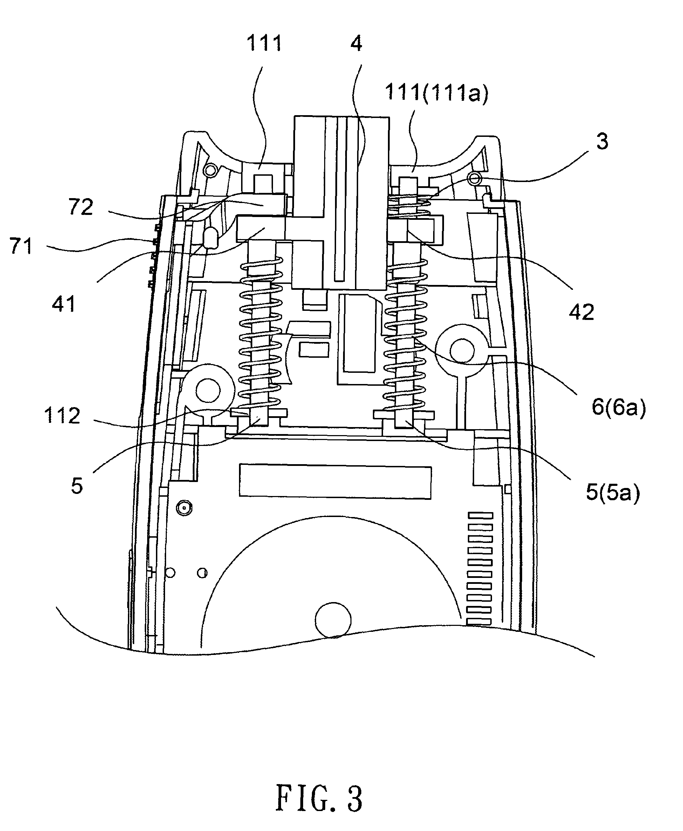

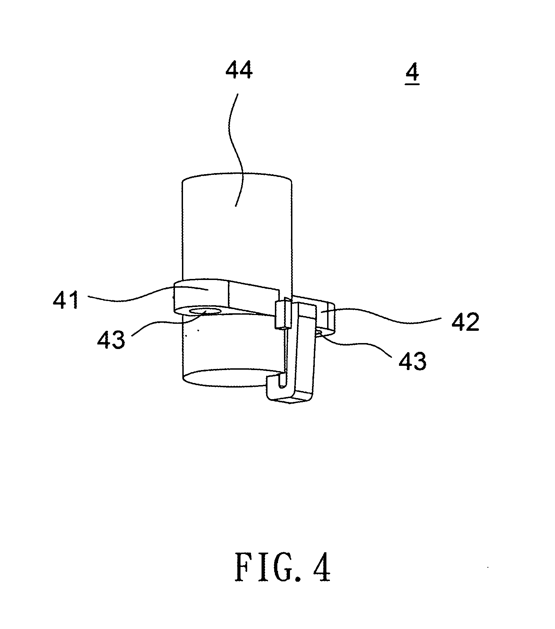

FIG. 1 shows an exploded view of a blood lancing device according to an embodiment of the present invention. FIG. 2A shows a schematic diagram for illustrating a lancet holder being disposed at a bottom cover of a blood lancing device according to an embodiment of the present invention. FIG. 2B shows a side view of a blood lancing device according to an embodiment of the present invention. FIG. 3 shows a grossly enlarged fragmentary view of the blood lancing device shown in FIG. 2A. As shown in FIGS. 1, 2A, 2B and 3, the blood lancing device 100 includes a lancet holder 4, and at least one slide rail 5 and a housing. The lancet holder 4 movable with respect to the housing is disposed in the housing and defines at least one through hole 43 (as shown in FIG. 4). The slide rail 5 is fixed in the housing, and it is inserted into the through hole 43 such that the lancet holder 4 may be moved along the slide rail 5. Since lateral (that is, the direction different from the extending direction of the slide rail 5) motion of the lancet holder 4 may be confined by the through hole 43 and the slide rail 5, the blood lancing device 100 of this embodiment has advantages of being not easy to be vibrated compared with the conventional blood lancing device.

In this embodiment, the housing includes a bottom cover 1 and top cover 8 respectively having a notch. When the bottom cover 1 and the top cover 8 are assembled together, the two notches form a through hole 18 from which the lancet holder 4 can extend out of the housing. The number of the slide rails 5 is two; each slide rail 5 extends along a longitudinal direction and is fixed on the bottom cover 1 in the housing. In another embodiment, the slide rails 5 may be fixed on the top bottom 8.

FIG. 4 shows a three dimensional view of a lancet holder according to an embodiment of the present invention. The lancet holder 4 includes a body portion 44 and at least one wing portion. In this embodiment, the at least one wing portion includes a first wing portion 41 and a second wing portion 42. The first wing portion 41 and the second wing portion 42 which respectively define a through hole 43 extend from two opposite sides of the body portion 44 so that the locations of the two wing portions 41 and 42 are symmetrical about the body portion 44. The two slide rails 5 are respectively inserted into the through holes 43, so that the lancet holder 4 may be moved forward or backward along the longitudinal direction of the slide rails 5. Since the locations of the two wing portions 41 and 42 are symmetrical about the body portion 44, vibration of the lancet holder 4 is not easy to occur when the lancet holder 4 moves. In one embodiment, the heights of the two wing portions 41 and 42 are smaller than that of the body portion 44 of the lancet holder 4. According to the above design, since the contact area between the slide rails 5 and the inside wells defining the through holes 43 of the two wing portions 41 and 42 is relatively small, the friction force during move of the lancet holder 4 may be reduced so that the lancet holder 4 can be easily moved forward or backward along the longitudinal direction of the slide rails 5. In one embodiment, the lancet holder 4 may not have the two wing portions 41 and 42 and only defines the through holes 43.

More specifically, according to conventional structure, the housing is used to define a containing space matching the shape of the lancet holder 4 such that the lancet holder 4 may be moved in the containing space. The contact area between the lancet holder 4 and the housing is relatively big, and the friction force is also bigger. Furthermore, in order to move the lancet holder 4 smoothly, the containing space must be set to be bigger than the volume of the lancet holder 4. As a result, the gap between is relatively big. However, the bigger the gap is, the easier the vibration of the lancet holder 4 occurs. On the contrary, in this embodiment, the contact area between the slide rails 5 and the inside wells defining the through holes 43 of the two wing portions 41 and 42 is smaller than the contact area between the lancet holder 4 and the housing according to conventional device. The cooperation of through holes 43 and the slide rails 5 can not only limit the lateral movement of the lancet holder 4, but also can reduce the friction force during move of the lancet holder 4. As a result, the lancet holder 4 may be moved smoothly and the vibration of the lancet holder 4 is not likely to occur.

In one embodiment, the blood lancing device 100 may further comprise at least one spring. The number of the springs in this embodiment is two. The two slide rails 5 are respectively inserted into the two springs 6 which is disposed between the first wing portion 41 and the bottom cover 1 and the second wing portion 42 and the bottom cover 1, respectively. In this embodiment, the bottom cover 1 has a first block 111 and a second block 112 extending from its bottom surface, and slide rails 5 are fixed between their respective first block 111 and second block 112. During deformation of each spring 6, one end of the springs 6 are respectively against the two wing portions 41 and 42 and the other end of the springs 6 are respectively against the second block 112 of the bottom cover 1.

In one embodiment, the blood lancing device 100 may further comprise a push structure 7. The push structure 7 includes a push-button 71 and an extending portion 72. The push-button 71 is exposed on the outside of the housing. In this embodiment, it is exposed on the side surface of the housing. It is preferred that a plurality of protruding blocks is formed on the push-button 71 to increase the friction force between a finger and the push-button 71. The extending portion 72 extends from the push-button 71 toward the inside of the housing, and defines a hole (not shown). The slide rails 5 are inserted into the hole of the extending portion 72 so that the extending portion 72 is disposed between the first wing portion 41 and the first block 111 and is adapted to be in contact with the first wing portion 41. As a result, the extending portion 72 and the spring 6 are disposed at the two opposite sides of the first wing portion 41. The user can push the push-button 71 to apply a force to the first wing portion 41 through the extending portion 72 so that the lancet holder 4 is moved from the first block 111l toward the second block 112 and then the spring 6 deforms due to compression from the extending portion 72. In one embodiment, the one inserted into the spring 6 may be the lancet holder 4 instead of the slide rail 5 so that the spring 6 suitable for providing a spring force to the lancet holder 4 is disposed between the lancet holder 4 and the housing. In one embodiment, one part of the body portion 44 may be inserted into the spring 6. The spring 6 may be in contact with the housing so that it can provide a spring force to the lancet holder 4. In one embodiment, the spring 6 is disposed in the way that it deforms due to the displacement of the lancet holder 4. As a result, in an operation state, the spring 6 can be released and give off energy to push the lancet holder 4 and let the lancet holder 4 move.

As shown in FIG. 3, the blood lancing device 100 may include a buffer spring 3. The other slide rail 5 (hereafter referred to as slide rail 5a) is inserted into the buffer spring 3 which is located between the other first block 111 (hereafter referred to as first block 111a) and the second wing portions 42. When the two springs 6 release the energy generated due to its deformation, they let the lancet holder 4 move from the second block 112 toward the first block 111. The buffer spring 3 can absorb the shock of the lancet holder 4 for the housing, so that the user can hold the blood lancing device 100 more securely.

A displaceable lancet (not shown) is disposed in the lancet holder 4 to puncture a skin of users and let they bleed. If the displaceable lancet was used, the users can abandon it to avoid contagion. In addition, the blood lancing device 100 according to an embodiment may further include a trigger button 9, a launching adjustment structure 10 for the lancet holder 4, a windows 11 for exposing the launching adjustment structure 10 outside, a battery cover 12 and a glucose strip hole13.

In one embodiment of the present invention, since the through holes 43 and the slide rails 5 can limit the lateral movement of the lancet holder 4, compared with the conventional device, the blood lancing device 100 according to an embodiment has the advantage that vibration of the lancet holder 4 is not easy to occur.

Although the preferred embodiments of the present invention has been fully described by way of examples with reference to the accompanying drawings, it should not be construed as any limitation on the implementation range of the invention. Various equivalent changes and modifications can be performed by those who are skilled in the art without deviating from the scope of the invention. The scope of the present invention is to be encompassed by the claims of the present invention. Any embodiment or claim of the present invention does not need to reach all the disclosed objects, advantages, and uniqueness of the invention. Besides, the abstract and the title are only used for assisting the search of the patent documentation and should not be construed as any limitation on the implementation range of the invention.

## Claims

1. A blood lancing device (100) comprising:
a housing;
a lancet holder (4) disposed in the housing and comprising a body portion for containing a lancet, ,
a push structure comprising a push-button being exposed on the outside of the housing and an extending portion extending from the push-button toward the inside of the housing, and
at least one slide rail (5) fixed in the housing,
**characterized in that** the lancet holder comprises at least one through hole (43) and at
least a wing portion extending from the body portion and defining the through hole, the slide rail (5) being inserted into the through hole (43) such that the lancet holder (4) is movable along the slide rail (5), and the extending portion defining a hole, wherein the slide rail is inserted into the hole so that the extending portion is in contact with the wing portion.

2. The blood lancing device (100) according to claim 1, wherein the height of the wing portion is smaller than that of the body portion (44).

3. The blood lancing device (100) according to claim 1 further comprising a resilient element, wherein the slide rail (5) is inserted into the resilient element, and the resilient element and the extending portion (72) are disposed at the two opposite sides of the wing portion.

4. The blood lancing device (100) according to claim 1 further comprising a resilient element, the resilient element being suitable for providing a spring force to the lancet holder (4) and being disposed between the lancet holder (4) and the housing.

5. The blood lancing device (100) according to claim 1, wherein
the at least one through hole (43) is formed as a first through hole (43) and a second through hole (43);
the at least one wing portion comprises a first wing portion (41) defining the first through hole (43) and a second wing portion (42) defining the second through hole (43); and
the at least one slide rail (5) comprises a first slide rail (5) inserted into the first through hole (43) and a second slide rail (5) inserted into the second through hole (43).

6. The blood lancing device (100) according to claim 5 further comprising a push structure (7), a first resilient element and a resilient component,
wherein the push structure (7) comprises a push-button (71) and an extending portion (72) wherein the push-button (71) is exposed on the outside of the housing, the extending portion (72) extends from the push-button (71) toward the inside of the housing and defines a hole, and the first slide rail (5) is inserted into the hole so that the extending portion (72) is in contact with the first wing portion (41),
wherein the first slide rail (5) is inserted into the first resilient element, and the first resilient element and the extending portion (72) are respectively disposed at the two opposite sides of the first wing portion (41),
and wherein the second slide rail (5) is inserted into the resilient component, and the resilient component is disposed between the second wing portion (42) and the housing.

7. The blood lancing device (100) according to claim 6 further comprising a second resilient element, wherein the second slide rail (5) is inserted into the second resilient element, and the second resilient element and the resilient component are disposed at the two opposite sides of the second wing portion (42).

8. The blood lancing device (100) according to claim 5, wherein the locations of the first wing portion (41) and the second wing portion (42) are symmetrical.

9. The blood lancing device according to claim 5, wherein the first wing portion and the second wing portion are disposed at the two opposite sides of the body portion.

10. The blood lancing device (100) according to claim 5, wherein the first slide rail (5) and the second slide rail (5) are substantially parallel.

11. The blood lancing device (100) according to claim 1 further comprising a resilient element, wherein the body portion (44) of the lancet holder (4) is inserted into the resilient element suitable for providing a spring force to the lancet holder (4).

## Patentansprüche

1. Blutlanzettenvorrichtung (100), die umfasst:
ein Gehäuse,
eine Lanzettenhalterung (4), die in dem Gehäuse angeordnet ist und einen Körperbereich zur Aufnahme einer Lanzette umfasst,
eine Drückstruktur, die einen Druckknopf, der an der Außenseite des Gehäuses exponiert ist, und einen Erstreckungsbereich umfasst, der sich von dem Druckknopf zu dem Inneren des Gehäuses hin erstreckt, und
mindestens eine Gleitschiene (5), die in dem Gehäuse befestigt ist,
**dadurch gekennzeichnet, dass** die Lanzettenhalterung mindestens ein Durchgangsloch (43) und mindestens einen Flügelbereich umfasst, der sich von dem Körperbereich erstreckt und das Durchgangsloch definiert,
wobei die Gleitschiene (5) durch das Durchgangsloch (43) so hindurch geführt ist, dass die Lanzettenhalterung (4) beweglich entlang der Gleitschiene (5) ist, und wobei der Erstreckungsbereich ein Loch definiert, in dem die Gleichschiene in das Loch so eingeführt ist, dass der Erstreckungsbereich in Kontakt mit dem Flügelbereich steht.

2. Blutlanzettenvorrichtung (100) nach Anspruch 1, bei der die Höhe des Flügelbereichs geringer ist als die des Körperbereichs (44).

3. Blutlanzettenvorrichtung (100) nach Anspruch 1, die ferner ein elastisches Element umfasst, wobei die Gleitschiene (5) in das elastische Element eingeführt ist und wobei das elastische Element und der Erstreckungsbereich (72) an den zwei gegenüberliegenden Seiten des Flügelbereichs angeordnet sind.

4. Blutlanzettenvorrichtung (100) nach Anspruch 1, die ferner ein elastisches Element umfasst, wobei das elastische Element geeignet ist, um eine Federkraft für die Lanzettenbalterung (4) bereitzustellen, und wobei das elastische Element zwischen der Lanzettenhalterung (4) und dem Gehäuse angeordnet ist.

5. Blutlattzettenvorrichtung (100) nach Anspruch 1, bei der das mindestens eine Durchgangsloch (43) als ein erstes Durchgangsloch (43) und als ein zweites Durchgangsloch (43) ausgebildet ist,
der mindestens eine Flügelbereich einen in dem ersten Durchgangsloch (43) vorgesehenen ersten Flügelbereich (41) und einen in dem zweiten Durchgangsloch (43) vorgesehenen zweiten Flügelbereich (42) umfasst und
die mindestens eine Gleitschiene (5) eine in das erste Durchgangsloch (43) eingeführte erste Gleitschiene (5) und eine in das zweite Durchgangsloch (43) eingeführte zweite Gleitschiene (5) umfasst.

6. Blutlanzettenvorrichtung (100) nach Anspruch 5, die ferner eine Drückstruktur (7), ein erstes elastisches Element und eine elastische Komponente umfasst,
wobei die Drückstruktur (7) einen Druckknopf (71) und einen Erstreckungsbereich (72) umfasst, wobei der Druckknopf (71) an der Außenseite des Gehäuses exponiert ist, wobei der Erstreckungsbereich (72) sich von dem Druckknopf (71) zu dem Inneren des Gehäuses hin erstreckt und ein Loch definiert und wobei die erste Gleitschiene (5) so in das Loch eingeführt ist, dass der Erstreckungsbereich (72) in Kontakt mit dem ersten Flügelbereich (41) steht,
wobei die erste Gleitschiene (5) in das erste elastische Element eingeführt ist und wobei das erste elastische Element und der Erstreckungsbereich (72) jeweils entsprechend an den zwei gegenüberliegenden Seiten des ersten Flügelbereichs (41) angeordnet sind, und wobei die zweite Gleitschiene (5) in die elastische Komponente eingeführt ist und wobei die elastische Komponente zwischen dem zweiten Flügelbereich (42) und dem Gehäuse angeordnet ist.

7. Blutlanzettenvorrichtung (100) nach Anspruch 6, die ferner ein zweites elastisches Element umfasst, wobei die zweite Gleitschiene (5) in das zweite elastische Element eingeführt ist und wobei das zweite elastische Element und die elastische Komponente an den zwei gegenüberliegenden Seiten des zweiten Flügelbereichs (42) angeordnet sind.

8. Blutlanzettenvorrichtung (100) nach Anspruch 5, bei der die Positionen des ersten Flügelbereichs (41) und des zweiten Flügelbereichs (42) symmetrisch sind.

9. Blutlanzettenvorrichtung (100) nach Anspruch 5, bei der der erste Flügelbereich und der zweite Flügelbereich an den zwei gegenüberliegenden Seiten des Körperbereichs angeordnet sind.

10. Blutlanzettenvorrichtung (100) nach Anspruch 5, bei der die erste Gleitschiene (5) und die zweite Gleitschiene (5) im Wesentlichen parallel sind.

11. Btutlanzettenvorrichtung (100) nach Anspruch 1, die ferner ein elastisches Element umfasst, wobei der Körperbereich (44) der Lanzettenhalterung (4) in das elastische Element eingesetzt ist, um eine Federkraft für die Lanzettenhalterung (4) bereitzustellen.

## Revendications

1. Autopiqueur de sang (100) comprenant :
un boîtier ;
un support de lancette (4) disposé dans le boîtier et comprenant une portion de corps pour contenir une lancette,
une structure de poussée comprenant un bouton-poussoir qui est exposé sur l'extérieur du boîtier et une portion d'extension qui s'étend du bouton-poussoir vers l'intérieur du boîtier et
au moins une glissière (5) fixée dans le boîtier,
**caractérisé en ce que** le support de lancette comprend au moins un trou de passage (43) et au moins une portion d'aile qui s'étend à partir de la portion de corps et qui définit le trou de passage, la glissière (5) étant insérée dans le trou de passage (43) de telle manière que le support de lancette (4) est mobile le long de la glissière (5) et la portion d'extension définissant un trou, la glissière étant insérée dans le trou de telle manière que la portion d'extension est en contact avec la portion d'aile.

2. Autopiqueur de sang (100) selon la revendication 1 dans lequel la hauteur de la portion d'aile est inférieure à celle de la portion de corps (44).

3. Autopiqueur de sang (100) selon la revendication 1 comprenant de plus un élément élastique, la glissière (5) étant insérée dans l'élément élastique et l'élément élastique et la portion d'extension (72) étant disposés aux deux côtés opposés de la portion d'aile.

4. Autopiqueur de sang (100) selon la revendication 1 comprenant de plus un élément élastique, l'élément élastique étant approprié pour fournir une force de ressort au support de lancette (4) et étant disposé entre le support de lancette (4) et le boîtier.

5. Autopiqueur de sang (100) selon la revendication 1 dans lequel :
le trou de passage qui existe au moins (43) est formé comme un premier trou de passage (43) et un second trou de passage (43) ;
la portion d'aile qui existe au moins comprend une première portion d'aile (41) qui définit le premier trou de passage (43) et une seconde portion d'aile (42) qui définit le second trou de passage (43) et
la glissière qui existe au moins (5) comprend une première glissière (5) insérée dans le premier trou de passage (43) et une seconde glissière (5) insérée dans le second trou de passage (43).

6. Autopiqueur de sang (100) selon la revendication 5 comprenant de plus une structure de poussée (7), un premier élément élastique et un composant élastique,
la structure de poussée (7) comprenant un bouton-poussoir (71) et une portion d'extension (72), le bouton-poussoir (71) étant exposé sur l'extérieur du boîtier, la portion d'extension (72) s'étendant du bouton-poussoir (71) vers l'intérieur du boîtier et définissant un trou et la première glissière (5) étant insérée dans le trou de telle manière que la portion d'extension (72) est en contact avec la première portion d'aile (71),
la première glissière (5) étant insérée dans le premier élément élastique et le premier élément élastique et la portion d'extension (72) étant disposés respectivement aux deux côtés opposés de la première portion d'aile (41)
et la seconde glissière (5) étant insérée dans le composant élastique et le composant élastique étant disposé entre la seconde portion d'aile (42) et le boîtier.

7. Autopiqueur de sang (100) selon la revendication 6 comprenant de plus un second élément élastique, la seconde glissière (5) étant insérée dans le second élément élastique et le second élément élastique et le composant élastique étant disposés aux deux côtés opposés de la seconde portion d'aile (42).

8. Autopiqueur de sang (100) selon la revendication 5 dans lequel les endroits de la première portion d'aile (41) et la seconde portion d'aile (42) sont symétriques.

9. Autopiqueur de sang (100) selon la revendication 5 dans lequel la première portion d'aile et la seconde portion d'aile sont disposées aux deux côtés opposés de la portion de corps.

10. Autopiqueur de sang (100) selon la revendication 5 dans lequel la première glissière (5) et la seconde glissière (5) sont substantiellement parallèles.

11. Autopiqueur de sang (100) selon la revendication 1 comprenant de plus un élément élastique, la portion de corps (44) du support de lancette (4) étant insérée dans l'élément élastique approprié pour fournir une force de ressort au support de lancette (4).
